# EUROPEAN PATENT APPLICATION

(11) **EP 3 287 100 A1**
(43) Date of publication of application: **28.02.2018**
(21) Application number: 16782486.1
(22) Date of filing: 02.03.2016
(51) Int. Cl.: A61F 2/24

(54) **HEART VALVE BIOPROSTHESIS AND MANUFACTURING METHOD THEREFOR**

(30) Priority: 20.04.2015 CN 201510189387
(71) Applicant: Hangzhou Jiahezhongbang Biotechnology Co., Ltd., Hangzhou, Zhejiang 310032 (CN)
(72) Inventor: YI, Dinghua, Hangzhou Zhejiang 310032 (CN); ZHANG, Hao, Hangzhou Zhejiang 310032 (CN); ZHAO, Yimin, Beijing 100097 (CN)
(74) Representative: Gong, Jinping
(86) International application number: PCT/CN2016/075359
(87) International publication number: WO 2016/169338

(57) **Abstract**

A heart valve bioprosthesis (100) and a manufacturingmethod thereof are provided. The heart valve bioprosthesis (100) comprises: a valve frame (1), a valve leaflet (2) and a valve auxiliary structure. The valve leaflet (2) is connected to the valve auxiliary structure, the valve auxiliary structure is connected to the valve frame (1). Both the valve leaflet (2) and the valve auxiliary structure are made of biomaterials. The valve frame (1) is integrally formed by an elastic material, and the valve auxiliary structure is preset with a suturing mark. The heart valve bioprosthesis improves the biocompatibility with the human body, reduces the total height of the valve, shortens the suturing time, and decreases the possibility of thrombus or bacterium attachment thereto. In addition, the heart valve bioprosthesis is made by simple processing, has an excellent compliance with the cardiac tissue, and has a prolonged valve service life.

## Description

### TECHNICAL FIELD

The present invention relates toa heart valve bioprosthesisused in a cardiovascular surgery (cardiac surgery).

### BACKGROUND OF THE INVENTION

In the medical field of cardiovascular surgery, if any lesion occurs to the valve inside the heart due to some reason(s) to cause hypofunction of the heart, in case of failure of internal medicine, it is generally necessary to use an implanting method by surgeons wherein the valve in lesion without its original function will be replaced with a valve prosthesis. Such heart valve prosthesis for the implantation surgery is currently classified into two types: the heart valve mechanical prosthesis and the heart valve bioprosthesis. The present invention concerns to the heart valve bioprosthesis. In term of whether a stent (valve frame) is present inside the valve, the heart valve bioprosthesis is classified into the valve with stent (valve frame) and the valve without stent (valve frame).

The valve without stent (valve frame) is made of only biomaterials or of only biomaterials plus the terylene cloth. The valve leaflet is made of a pericardium of a cow/ox, a horse, or a pig. The covering and the suturing edge (ring) is made of a pericardium of a cow/ox or a horse and/or the terylene cloth. The valve without stent has the following advantages: due to the obsence of stent, it has a outstanding compliance with the heart after implantation into the heart; the various portions of the valve leaflet bear minimized stresses and a relatively small damage, thus having a long life. It has the following disadvantages: still due to the obsence of stent (valve frame), it is quite difficult for positioning during an implantation operation, necessary for the surgeon to have a high skill level and inconvenient for the surgeon to perform precise implantation.Thus, it is seldom used and developed.

The valve with stent (valve frame) is generally made of three materials: metals or plastics, biomaterials, and the terylene cloth (also called polyester fiber or polyethylene terephthalate or PET, hereinafter asterylene for short). Currently, the valve bioprosthesiswith stent (valve frame) accounts for 95% of the valve bioprostheses. The valve with stent has the following advantages: due to the presence of stent (valve frame), it is convenient for positioning in an implantation operation, facilitating precise implantation by the surgeon. However, the prior valve with stent is traditionally made of three materials: the biomaterial for making the valve leaflet, the metal (alloy) or polymer material for making the valve frame, and the terylene cloth for making the covering and the suturing edge (ring). Such type of valve has the following disadvantages: 1) the terylene cloth, after implantation into the heart, being exposed to the blood to cause a relatively high possibility of thrombus or increased bacterium attachment thereto; 2) the valve frame, as a structure formed by combination of the metal valve frame wire and the metal plate valve seat, being high in hardness but poor in elasticity and compliance, and the valve leaflet being prone to strain; 3) the valve frame, as a structure formed by combination of the metal valve frame wire and the metal plate valve seat, being complicated in manufacturing processing and structure, and the valve frame wire having welding points and a sleeve tube to cause a break risk; and 4) the valve having a relatively large total height such that after implantation into the heart, the aortic valveis prone to block the blood flow at the opening of the coronary artery and the mitral valve is prone to impede the movement of the original sub-valvular tissue.

The valve, either with or without stent, uses the terylene clothto make the covering and the suturing edge due to the followingreasons: on one hand, the terylene cloth has an excellent chemicalinertness, sterilization resistance, good mechanical properties, a low water absorption and a relatively good biocompatibility, specifically, the terylene cloth can be sterilized by traditional technologies, without changing its inherent nature, and has many advantages, such as high strength, good elasticity, good wear resistance, good fatigue resistance, and good dimensional stability, etc. Also, the terylene cloth has a relatively good chemicalinertness, can induce tissue growth, has a good fiberization reactivity, and has been proved safe during the history of implantation of more than 50 years; on the other hand, as it has a use history of decades, those skilled in the art, when designing the covering and the suturing edge, will generally use the terylene cloth.

In addition, for any type of valve bioprosthesis, during manufacturing, various portions thereof are sutured manually. The suturing points and the suturing spacing depend on the experiences of the technical operators only, and it is difficult for suturing quality controlling and training.

As the traditional heart valve bioprosthesis is outmoded in design concept and is complicated in structure, there are many disadvantages in use and function. Therefore, the present invention is designed and developed.

### SUMMARY OF THE INVENTION

The objective of the present invention is to provide a heart valve bioprosthesis which can decrease thrombus or bacterium attachment thereto. Another objective of the present invention is to solve the technical problems of complicated structure, over-hard valve frame, and poor compliance with heart. Another objective of the present invention is to solve the problem of suturing by experience and without standardization during manufacturing of a traditional valve bioprosthesis.

In order to achieve the above objectives, according to an aspect of the present invention, a heart valve bioprosthesisis provided.The heart valve bioprosthesis comprises: a valve frame, a valve leaflet and a valve auxiliary structure, wherein the valve leaflet is connected to the valve auxiliary structure, the valve auxiliary structure is connected to the valve frame, and the valve leaflet and the valve auxiliary structure are made of biomaterials.

In an embodiment, the valve auxiliary structure may comprise a suturing edge and a covering, the valve leaflet is connected to the covering, and the covering and the suturing edge are fixed directly or indirectly on the valve frame.

Preferably,the valve leaflet is sutured to the covering. Preferably, the covering is fixed to the valve frame, and the suturing edge is connected to the covering.

Preferably, the valve frame comprises a valve ring and a valve ridge which are integrally formed, and the suturing edge is sutured to an outer side edge of the valve ring.

In an embodiment, the covering may comprise a valve frame inner side covering and a valve frame outer side covering, wherein the valve leaflet is connected to the inner side covering, and the valve frame is placed within a space surrounded by the inner side covering and the outer side covering.

The inner side covering and the outer side covering are sutured together to form the upper and lower edge coverings with valve frame.

Preferably, the valve leaflet is sutured to a top edge of the inner side covering and a top edge of the outer side covering is sutured to the top edge of the inner side covering to form the upper edge covering; and a lower edge of the inner side covering is sutured to a lower edge of the outer side covering to form the lower edge covering, thus enclosing the valve frame into a space surrounded by the inner side covering, the outer side covering as well as the upper and lower edge coverings formed by suturing them together.

In an embodiment, the inner side covering is provided at its upper portion with three notches matching in shape with the valve leaflets, and has a lower portion with a profile matching in shape with the valve ring of the valve frame. Between any two notches, a protrusion is provided, matching in shape with the valve ridge of the valve frame. Further, on two sides of the inner side covering, two protrusions are provided, respectively. These two protrusions, if combined, may form a shape matching with the shape of one valve ridge of the valve frame.

In an embodiment, the outer side covering is provided at its upper portion with three protrusions matching in shape with the valve ridge of the valve frame, and has a lower portion with a profile matching in shape with the valve ring of the valve frame. Between any two protrusions, a notch is provided, matching in shape with the valve leaflet. Further, on two sides of the outer side covering, two notches are provided, respectively. These two notches, if combined, may form a shape matching with the shape of one valve leaflet.

In an embodiment, the inner side covering, the outer side covering and the suturing edge are integrally formed.

In an embodiment, the outer side covering and the suturing edge are integrally formed.

Preferably, each of the valve leaflet, the suturing edge and the covering is preset with a suturing mark such that in suturing, the heart valve bioprosthesis is prepared by suturing along the suturing marks.

In an embodiment, the valve frame may be integrally formed.

In an embodiment, the valve frame may be made of an elastic material.

In an embodiment, a distance between two adjacent ones of the suturing marks is 0.5 - 3mm.

In an embodiment, the valve frame may be made of polyformaldehyde (POM), polyetheretherketone (PEET), polysulfone (PSF), Co-based alloy, Ti-based alloy, or Ni-Ti alloy.

In an embodiment, the valve auxiliary structure may be made of an animalpericardium, and the valve leaflet may be made of an animalpericardium or of an aortic valve leaflet of a pig.

Preferably, the above animalpericardium is a pericardium of a cow/ox, a horse, a pig, a sheep/goat, a donkey, or a mule.

The suturing mark may be a suturing hole, a suturing point, or other marks easy to identify.

Preferably, the suturing mark is preset in any position necessary for suturing in manufacturing of the heart valve bioprosthesis.

Preferably, the suturing marks are preset along the periphery of the inner side covering, the periphery of the outer side covering as well as the periphery of the suturing edge.

According to another aspect of the present invention, a heart valve bioprosthesis is provided.The heart valve bioprosthesis comprises: a valve frame, a valve leaflet and a valve auxiliary structure, the valve leaflet is connected to the valve auxiliary structure, the valve auxiliary structure is connected to the valve frame, wherein the valve auxiliary structure is preset with a suturing mark such that in suturing, the heart valve bioprosthesis is prepared by suturing along the suturing mark.

In an embodiment, the valve frame may be integrally formed.

In an embodiment, the valve frame may be made of an elastic material.

In an embodiment, the valve auxiliary structure may comprise a suturing edge and a covering, the valve leaflet is connected to the covering, and the covering and the suturing edge are fixed directly or indirectly on the valve frame.

In an embodiment, the covering may comprise an inner side covering and an outer side covering, wherein the valve leaflet is connected to the inner side covering, and the valve frame is placed within a space surrounded by the inner side covering and the outer side covering.

Preferably, each of the valve leaflet, the suturing edge and the covering is preset with a suturing mark such that in suturing, the heart valve bioprosthesis is prepared by suturing along the suturing mark.

In an embodiment, the valve leaflet and the valve auxiliary structure may be made of biomaterials.

In an embodiment, the elastic material may be polyformaldehyde (POM), polyetheretherketone (PEET), polysulfone (PSF), Co-based alloy, Ti-based alloy, or Ni-Ti alloy.

The suturing mark may be a suturing hole, a suturing point, or other marks easy to identify.

According to a further aspect of the present invention, a manufacturing method of a heart valve bioprosthesis is provided.The manufacturing method comprises the following steps:
a) providing a valve frame, a valve leaflet, a suturing edge and a covering;
b) presetting suturing marks to the valve leaflet, the suturing edge and the covering;
c) along the suturing marks, suturing the valve leaflet to the covering, and fixing the sutured valve leaflet and covering to the valve frame;
d) fixing the suturing edge to the valve frame to form the heart valve bioprosthesis.

In an embodiment, the covering comprises an inner side covering and an outer side covering, and the step c) comprises: suturing the valve leaflet to the inner side covering and suturing the outer side covering to the inner side covering to form upper and lower edge coverings.

Preferably, the step c) comprises: placing the valve frame into a space surrounded by the inner side covering and the outer side covering.

Preferably, the step c) comprises: suturing the valve leaflet to a top edge of the inner side covering and suturing a top edge of the outer side covering to the top edge of the inner side covering to form the upper edge covering; and the step c) comprises: suturing a lower edge of the inner side covering to a lower edge of the outer side covering to form the lower edge covering, thus enclosing the valve frame into a space surrounded by the inner side covering, the outer side covering as well as the upper and lower edge coverings formed by suturing them together.

Preferably, the valve frame comprises a valve ring and a valve ridge which are integrally formed; and the step d) comprises: suturing the suturing edge to an outer side edge of the valve ring.

Preferably, the valve leaflet is sutured to the covering by a method of reciprocating suturing, and/or the upper edges of the inner and outer side coverings are sutured together by a method of edge locking suturing.

In the method, the suturing mark may be a suturing hole, a suturing point, or other marks easy to identify.

The heart valve bioprosthesis of the present invention decreases the possibility of thrombus or bacterium attachment thereto, is made by simple processing, has an excellent compliance with the cardiac tissue, and has a prolonged valve service life.

### DESCRIPTION OF THE DRAWINGS

Figure 1 is an exploded perspective view of a heart valve bioprosthesis according to an embodiment of the present invention.
Figures 2-10 show a manufacturing method of a heart valve bioprosthesis according to an embodiment of the present invention.
Figures 11 and 12 show structural diagrams of the inner and outer side coverings, respectively, of a heart valve bioprosthesis according to a second embodiment of the present invention.
Figures 13-17 show the manufacturing steps of the heart valve bioprosthesis of the embodiments shown in figures 11 and 12.
Figures 18 and 19 show structural diagrams of the inner and outer side coverings, respectively, of a heart valve bioprosthesis according to a third embodiment of the present invention.
Figures 20-24 show the manufacturing steps of the heart valve bioprosthesis of the embodiments shown in figures 18 and 19.
Figure 25 shows a structural diagram of the covering(s) of a heart valve bioprosthesis according to a fourth embodiment of the present invention.
Figures 26-30 show the manufacturing steps of the heart valve bioprosthesis of the embodiment shown in figure 25.

### DESCRIPTION OF EXEMPLARY EMBODIMENTS

Hereinafter, the preferred embodiments of the present invention will be explained in detail with reference to the accompanying drawings, to make the objective(s), characteristics and advantages of the present invention better understood. It should be understood that the embodiments shown in the figures are not to limit the scope of the present invention; instead, they are provided only to explain the substantial spirit of the technical solutions of the present invention.

Explanation for the terms:
Valve frame: it is in a shape of an irregular circle ring and consists of a valve ring (in a wave-like shape or a horizontal shape) and a valve ridge (three ridged bulges); in a traditional heart valve bioprosthesis, the valve frame is formed by combination of a valve frame wire and a valve seat, wherein the valve frame wire is in a shape of an irregular circle ring and is formed by the valve ridge (three ridged bulges), while the valve seat is a circle ring in a wave-like shape or a horizontal shape; in the heart valve bioprosthesis of the present invention, the valve frame is integrally formed.

Valve leaflet: it is formed by suturing with an animalpericardium (a pericardium of a cow/ox, a horse, a pig, a sheep/goat, a donkey, or a mule) and/or with an aortic valve leaflet of a pig.

Suturing edge (or the so-called suturing ring): the suturing edge is used to fix the heart valve to the cardiac tissue;it is provided to the outer edge of the valve frame, is generally formed by suturing, and is generally in a ring shape (or it may be in other shape), thus sometimes being called as the suturing ring.

Covering(s): it is a material sutured to the inner and outer edges and upper and lower edges of the valve frame.

Valve auxiliary structure: it is a general term of the suturing edge(s) and the covering(s).

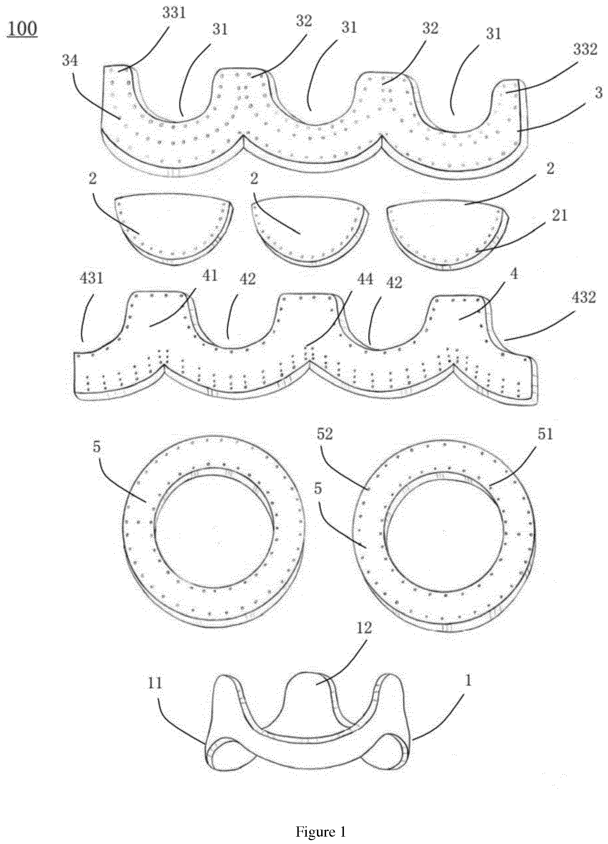

Figure 1 is an exploded perspective view of a heart valve bioprosthesis according to an embodiment of the present invention.As shown in figure 1, the heart valve bioprosthesis 100 comprises: a valve frame 1, a valve leaflet 2, an inner side covering 3, an outer side covering 4, and a suturing ring 5, wherein the valve leaflet 2 is sutured to the inner side covering 3, the outer side covering 4 is sutured to the inner side covering 3, and then the valve leaflet 2, the inner side covering 3 and the outer side covering 4 are together mounted to the valve frame 1 and sutured to the valve frame 1, that is, the valve frame is placed within a space surrounded by the inner side covering 3 and the outer side covering 4. Finally, the suturing ring 5 is fixed on the valve frame, thus forming the complete heart valve bioprosthesis. Herein, the inner side covering 3, the outer side covering 4 and the suturing ring 5 may be generally called as the valve auxiliary structure. The inner side covering 3 and the outer side covering 4 may be generally called as the covering. Herein, the suturing ring may be fixed to the valve frame by suturing to the inner side covering and/or the outer side covering, or may be sutured directly to the valve frame.

The valve frame 1 comprises a valve ring 11 and a valve ridge 12 which are integrally formed, wherein the valve ring 11 is in a shape of an irregular circle ring and the valve ridge 12 comprises three ridged bulges. Preferably, the valve frame 1 is integrally formed by an elastic material. Preferably, the valve frame 1 is provided thereon with no hole or slot structure. More preferably, the valve frame is made of polyformaldehyde (POM), polyetheretherketone (PEET), polysulfone (PSF), Co-based alloy, Ti-based alloy, or Ni-Ti alloy.

The valve leaflet 2 is substantially in a semi-circle shape and is preset thereon with a suturing hole 21 used for suturing the valve leaflet 2 to the inner side covering 3. A heart valve generally comprises three valve leaflets 2. The valve leaflet is made of a biomaterial. Preferably, the valve auxiliary structure is made of an animalpericardium, and the valve leaflet is made of an animalpericardium or of an aortic valve leaflet of a pig. The animalpericardium is a pericardium of a cow/ox, a horse, a pig, a sheep/goat, a donkey, or a mule, or any suitable animal.

The inner side covering 3 is provided at its upper portion with three notches 31 matchingin shape with the valve leaflets 2, and has a lower portion with a profile matching in shape with the valve ring of the valve frame. Between any two notches 31, a protrusion 32 is provided, matching in shape with the valve ridge of the valve frame. Further, on two sides of the inner side covering 3, a protrusion 331 and a protrusion 332 are provided, respectively. The protrusions 331 and 332, if combined, may form a shape matching with the shape of one valve ridge of the valve frame. The inner side covering is preset with a suturing hole 34 used for suturing it together with the valve leaflet and the outer side covering 4. In the present embodiment, the suturing holes are arranged along the periphery of the inner side covering, and a row of suturing holes is further provided in the middle portion of the inner side covering. However, it should be understood that the suturing holes 34 may be preset on any site of the inner side covering necessary for suturing in manufacturing of the heart valve bioprosthesis. The inner side covering is made of a biomaterial. Preferably, the biomaterial is an animalpericardium, such as a pericardium of a cow/ox, a horse, a pig, a sheep/goat, a donkey, or a mule, or any suitable animal.

The outer side covering 4 is provided at its upper portion with three protrusions 41 matching in shape with the valve ridge of the valve frame, and has a lower portion with a profile matching in shape with the valve ring of the valve frame. Between any two protrusions 41, a notch 42 is provided, matching in shape with the valve leaflet 2. Further, on two sides of the outer side covering 4, a notch 431 and a notch 432 are provided, respectively. The notches 431 and 432, if combined, may form a shape matching with the shape of one valve leaflet 2. The outer side covering is preset with a suturing hole 44 used for suturing it together with the inner side covering 4. In the present embodiment, the suturing holes are arranged along the periphery of the outer side covering, and a row of suturing holes is further provided in the middle portion of the inner side covering. However, it should be understood that the suturing holes 44 may be preset on any site of the outer side covering necessary for suturing in manufacturing of the heart valve bioprosthesis. The outer side covering 4 is made of a biomaterial. Preferably, the biomaterial is an animalpericardium, such as a pericardium of a cow/ox, a horse, a pig, a sheep/goat, a donkey, or a mule, or any suitable animal.

The suturing ring 5 is in a shape of a circle ring, and is preset at its inner edge and outer edge with pre-suturing holes 51 and 52, wherein the pre-suturing holes 51 on the inner edge are used for suturing it to the outer covering while the pre-suturing holes 52 on the outer edge are used for suturing the two suturing rings 5 together. The suturing ring 5 is made of a biomaterial. Preferably, the biomaterial is an animalpericardium, such as a pericardium of a cow/ox, a horse, a pig, a sheep/goat, a donkey, or a mule, or any suitable animal.

It should be pointed out that the pre-suturing holes provided on the valve leaflet 2, the inner side covering 3, the outer side covering 4 and the suturing ring 5 may be substituted with other suturing marks, such as colored points or other marks provided in or near the position to be sutured. The operator, when suturing the heart valve bioprosthesis, may perform suturing along these suturing marks. Preferably, a distance between two adjacent ones of the suturing marks is 0.5 - 3mm, more preferably 1.4 - 2mm. The manner of providing the suturing marks may comprise the laser drilling, the jig &fixture drilling (similar in principle to the paper hole puncher), etc. In addition, the suturing marks may be provided in any position necessary for suturing, on one or more of the valve leaflet 2, the inner side covering 3, the outer side covering 4 and the suturing ring 5, without being limited to the position(s) disclosed herein.

In comparison with the heart valve bioprosthesis in the prior art, the heart valve bioprosthesis of the present invention uses the special valve frame and biomaterial covering and presets the suturing marks, and thus improves the compliance with the heart and the biocompatibility with the human body, reduces the total height of the valve, shortens the suturing time, and decreases the possibility of thrombus or bacterium attachment thereto, as detailed in the following Table 1.

Table 1 is to compare several examples of the heart valve bioprosthesis of the present invention with those in the prior art.

| Examples | | Valve type | Valve frame material | Covering material | Valve leaflet material | Whether to provide pre-sutur ing holes | Whether to use an integrally formed valve frame | Total valve height (mm) | Suturing time (hour) | thrombus or bacterium attachment |
|---|---|---|---|---|---|---|---|---|---|---|
| The present invention | Example 1 | A21 aortic valve | POM | cow/oxperica rdium | cow/oxpericardi um | yes | yes | 12 | 2.5 | Level 3 |
| | Example 2 | A21 aortic valve | PEET | horse pericardium | cow/oxpericardi um | yes | yes | 12 | 2.5 | Level 3 |
| | Example 3 | M25 mitral valve | Ni-Ti alloy | pig pericardium | pig aortic valve leaflet | yes | yes | 14.5 | 2.5 | Level 3 |
| The prior art | Comparison example 1 | A21 aortic valve | Stainless steel | terylene | cow/oxpericardi um | no | no | 14-15 | 4 | Level 1 |
| | Comparison example 2 | A21 aortic valve | plastic | terylene | horse pericardium | no | yes | 14-15 | 4 | Level 1 |
| | Comparison example 3 | M25 mitral valve | plastic | terylene | cow/oxpericardi um | no | no | 15-18 | 4 | Level 1 |

| | | | | | | | | | | |
|---|---|---|---|---|---|---|---|---|---|---|
| Note: the levels for thrombus or bacterium attachment are classified as below: Level 1 refers to "high possibility of bacterium attachmentor thrombusoccurrence, with a proportion of 1.0% or less". Level 2 refers to "low possibility of bacterium attachmentor thrombusoccurrence, with a proportion of 0.1% or less". Level 3 refers to "very low possibility of bacterium attachmentor thrombusoccurrence, with a proportion of 0.01% or less". | | | | | | | | | | |

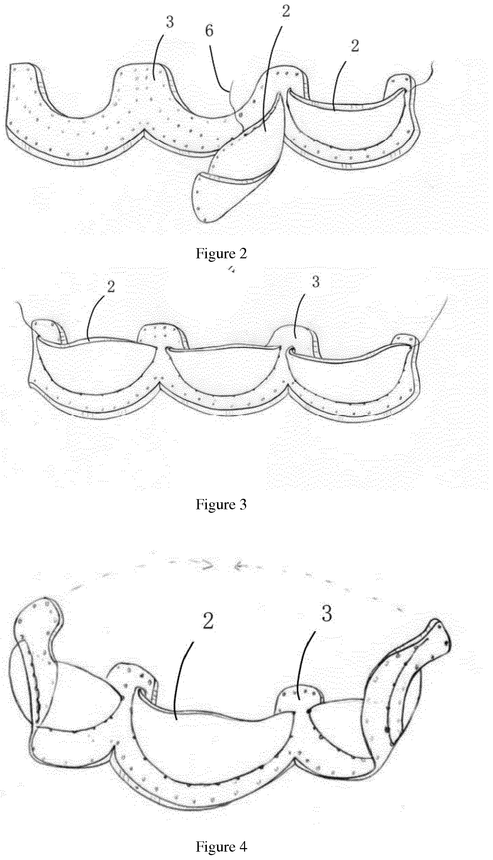

Figures 2-10 show a manufacturing method of a heart valve bioprosthesisaccording to an embodiment of the present invention.The method of the present invention comprises the following steps:
a) providing a valve frame, a valve leaflet, a suturing edge and a covering;
b) presetting suturing holes to the valve leaflet, the suturing edge and the covering;
c) along the suturing holes, suturing the valve leaflet to the covering;
d) fixing the sutured valve leaflet and the sutured covering to the valve frame;
e) fixing the suturing edge to the valve frame to form the heart valve bioprosthesis.

As shown in figures 2-10, in the present embodiment, the valve frame 1 is an integrally formed part (formed into one piece), and there are three valve leaflets. The covering consists of an inner side covering 3 and an outer side covering 4. There are two suturing rings 5. It should be understood that the manufacturing method of the present invention may be also suitable to other similar heart valve bioprosthesis. Themanufacturing method of the present invention follows a basic principle of presetting suturing holes on the valve leaflet, the covering and the suturing edge such that in manufacturing of the heart valve, it is necessary to just perform suturing along the preset suturing holes, thus being simple for manufacturing and easy for product standardization.

Specifically, first, the suturing line 6 is used to suture the valve leaflets 2, one by one, to the inner side covering 3, along the suturing holes, that is, the valve leaflets are sutured to the top edge of the inner side covering, as shown in figures 2 and 3. During the suturing, preferably, the valve leaflets are sutured to the inner side covering by a method of reciprocating suturing. Next, the inner side covering 3 is bent/curled to a barrel shape and its two ends are sutured together, as shown in figures 4-5.

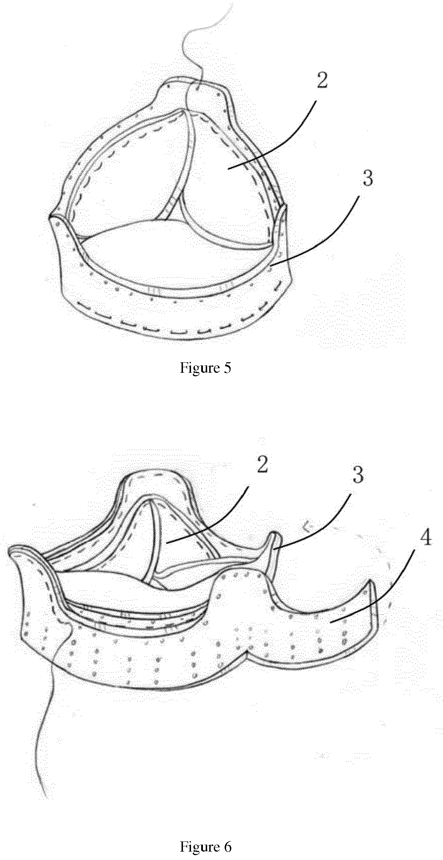

Then, the suturing line 6 is used to suture the outer side covering 4 to the inner side covering 3, along the suturing holes, that is, the tope edge of the outer side covering 4 is sutured to the top edge of the inner side covering 3, as shown in figure 6. During suturing the outer side covering 4 to the inner side covering 3, preferably, the upper edges of the inner and outer side coverings are sutured together by a method of edge locking suturing.

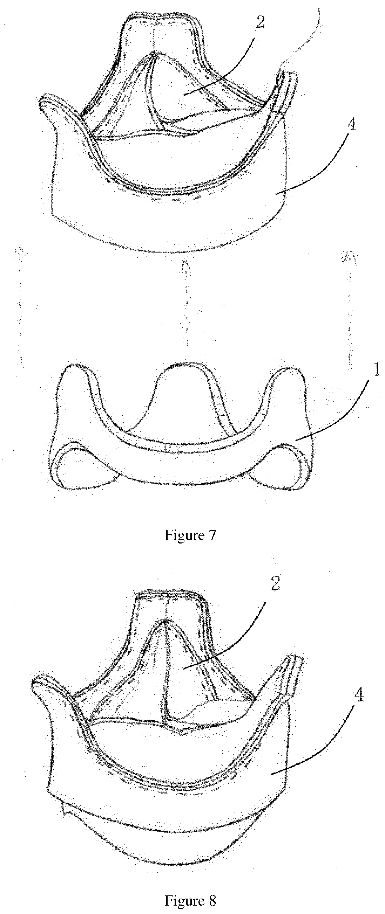

Next, the sutured valve leaflet 2, the inner side covering 3 and the outer side covering 4 are together mounted to the valve frame 1 such that the valve frame 1 is placed within a space surrounded by the inner side covering 3 and the outer side covering 4. Then, the lower edges of the inner and outer side coverings are sutured together along the suturing holes, as shown in figures 7-9. During such suturing, preferably, the lower edges of the inner and outer side coverings are sutured together by a method of edge locking suturing.

Finally, the suturing line 6 is used to suture the suturing ring 5 to the valve ring outer side edge of the valve frame, along the suturing holes. Specifically, the inner edges of the two suturing rings are sutured to the outer side covering by a method of S type suturing, and then the outer edges of the two suturing rings are sutured together by a method of spiral suturing. After the suturing rings are sutured, a certain outer edge/margin is retained to facilitate suturing it into the heart or the aorta in a valve replacement operation.

Therefore, the manufacturing of the heart valve bioprosthesis of the present invention is completed.

It should be pointed out that the structure and the shape of the heart valve bioprosthesis of the present invention are not limited to the above structure and shape. For example, the inner and outer side coverings may be integrally formed, then form the inner side covering and the outer side covering after being sutured to the valve frame.

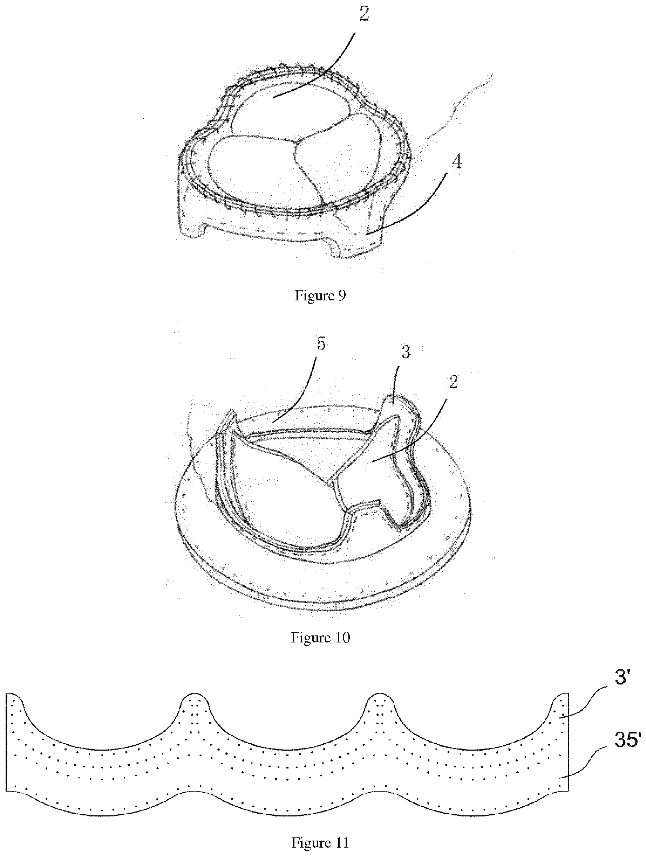

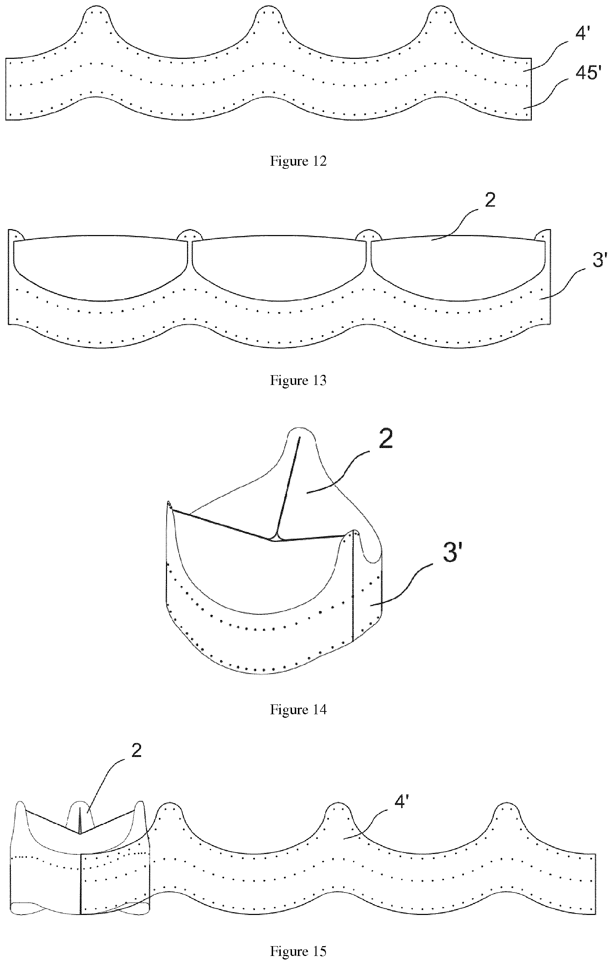

Figures 11 and 12 show structural diagrams of the inner side covering 3' and the outer side covering 4', respectively, of a heart valve bioprosthesis according to a second embodiment of the present invention.The present embodiment differs from that shown in figure 1 in that in the present embodiment, the suturing ring is integrally formed on the inner side covering and the outer side covering. Specifically, the lower edges 35' and 45' of the inner and outer side coverings are extended such that after the suturing, the lower edges 35' and 45' of the inner and outer side coverings 3' and 4' form the suturing edge. Accordingly, the suturing ring is not provided separately in the present embodiment, which is not as the one in figure 1. In the present embodiment, the structures of the upper portions of the inner and outer side coverings 3' and 4' and the structure of the valve frame are same as those of the embodiment of figure 1, and thus will not be described in detail.

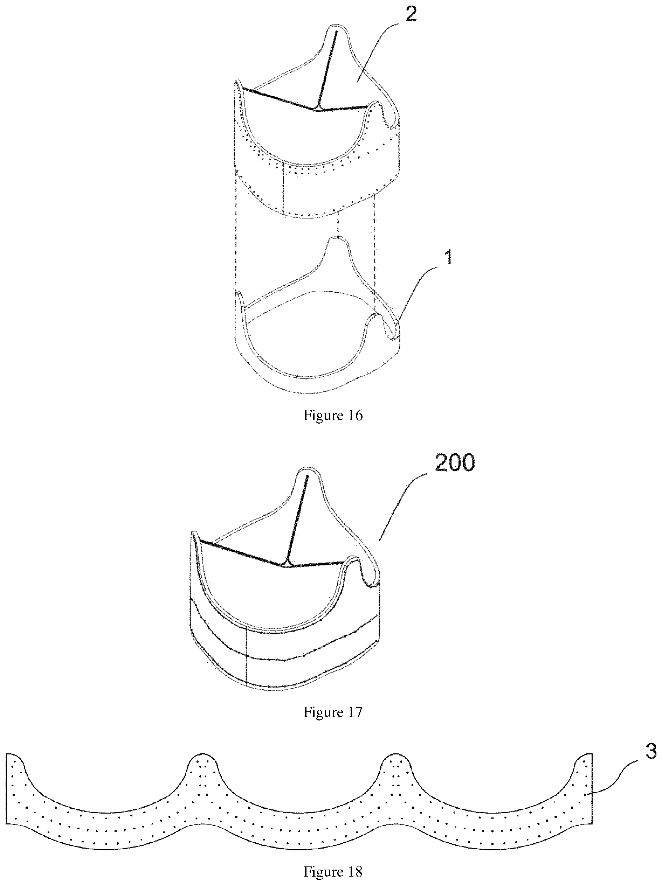

In the present embodiment, in suturing, as shown in figures 13-17, the suturing steps are set forth as below:
1) suturing the valve leaflet 2 to the inner side covering 3' by a method of reciprocating suturing, as shown in figure 13.
2) bending / curling the inner side covering 3' to a barrel shape and suturing its two ends together, as shown in figure 14.
3) suturing the upper edges of the inner and outer side coverings 3' and 4' together by a method of edge locking suturing, as shown in figure 15.
4) placing the valve frame 1 between the inner and outer side coverings, as shown in figure 16.
5) suturing the lower edges of the inner and outer side coverings 3' and 4' together by a method of edge locking suturing, to form the heart valve bioprosthesis 200, as shown in figure 17.

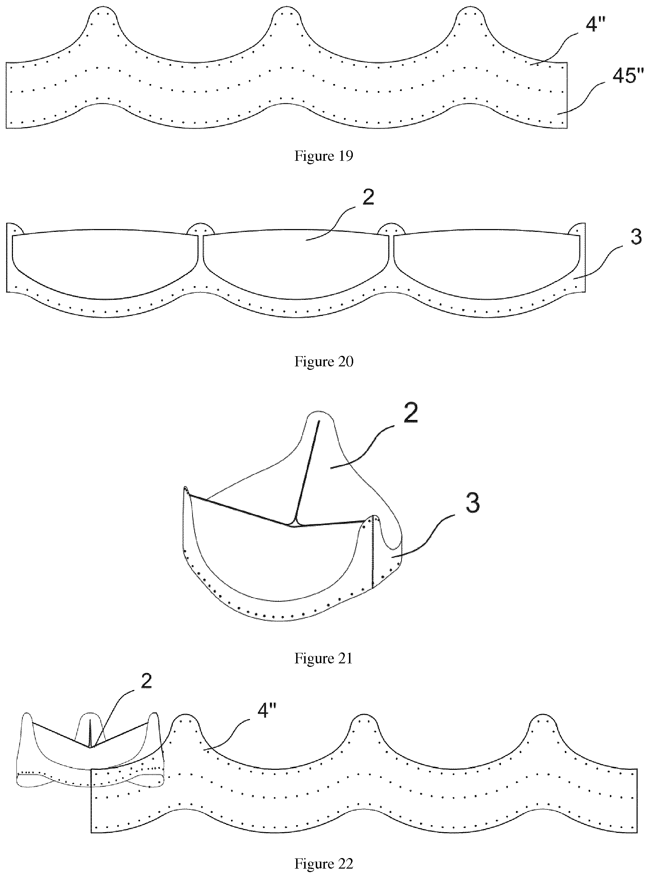

Figures 18 and 19 show structural diagrams of the inner side covering 3 and the outer side coverings 4", respectively, of a heart valve bioprosthesis according to a third embodiment of the present invention. The present embodiment differs from that shown in figure 1 in that in the present embodiment, the suturing ring is integrally formed on the outer side covering 4". Specifically, the lower edge 45" of the outer side covering 4" is extended such that after the suturing, the lower edge 45" of the outer side covering 4" forms the suturing edge. Accordingly, the suturing ringis not provided separately in the present embodiment, which is not as the one in figure 1. In the present embodiment, the structure of the inner side covering 3 is same as that of the inner side covering 3 in figure 1, and the structure of the upper portion of the outer side covering 4" and the structure of the valve frame 1 are same as those of the embodiment of figure 1, and thus will not be described in detail.

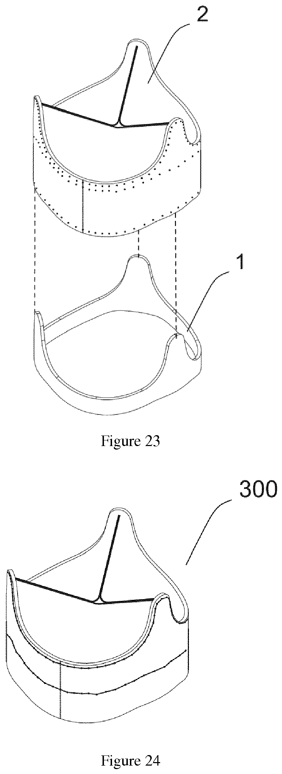

In the present embodiment, in suturing, as shown in figures 20-24, the suturing steps are set forth as below:
1) suturing the valve leaflet 2 to the inner side covering 3 by a method of reciprocating suturing, as shown in figure 20.
2) bending / curling the inner side covering 3 to a barrel shape and suturing its two ends together, as shown in figure 21.
3) suturing the upper edges of the inner and outer side coverings 3 and 4" together by a method of edge locking suturing, as shown in figure 22.
4) placing the valve frame 1 between the inner and outer side coverings, as shown in figure 23.
5) suturing the lower edges of the inner and outer side coverings 3 and 4" together by a method of edge locking suturing, to form the heart valve bioprosthesis 300, as shown in figure 24.

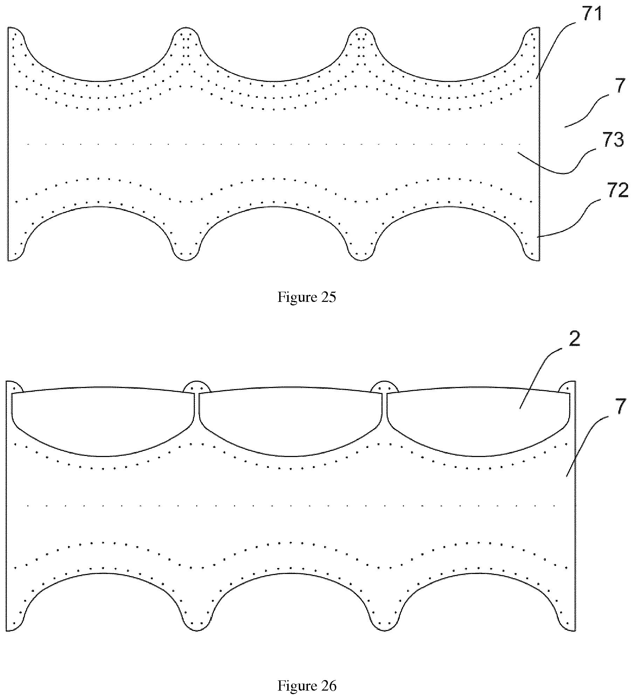

Figure 25 shows a structural diagram of the integrally formed covering 7 of a heart valve bioprosthesis according to a fourth embodiment of the present invention.The present embodiment differs from that shown in figure 1 in that in the present embodiment, the inner side covering, the outer side covering and the suturing ring are integrally formed into one piece. Specifically, in the present embodiment, the upper portion 71 of the covering 7 is the inner side covering, and its structure is same as the inner side coveringshown in figure 1 and thus will not be described in detail herein. The lower portion 72 of the covering 7 is the outer side covering, and its structure is same as the outer side coveringshown in figure 1 and thus will not be described in detail herein. The middle portion 73 of the covering 7, after suturing, forms the suturing edge. In the present embodiment, the structure of the valve frame is same as that of the valve frame of the embodiment of figure 1 and thus will not be described in detail herein.

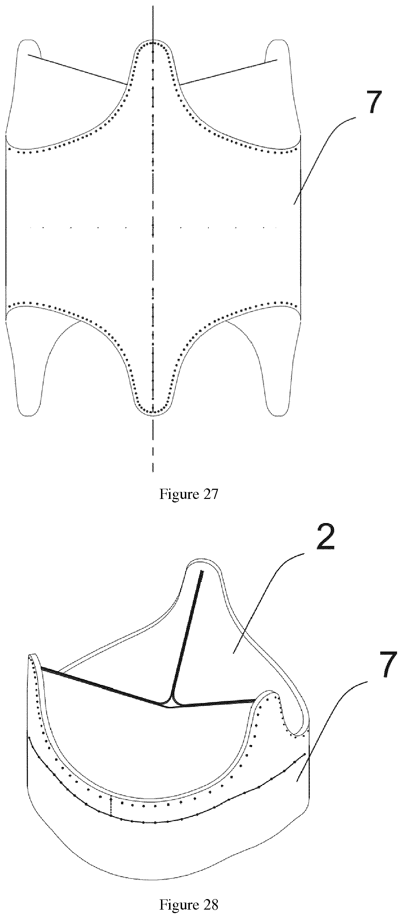

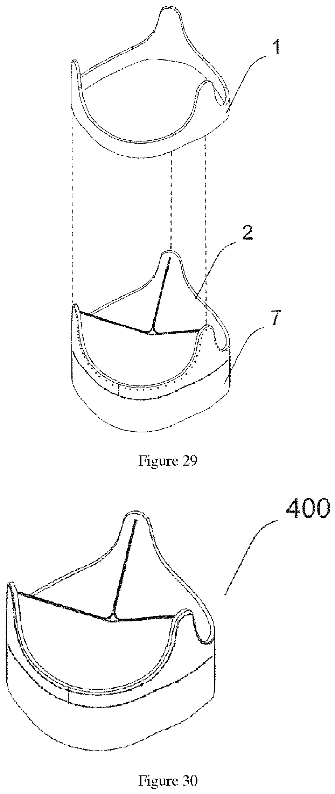

In the present embodiment, in suturing, as shown in figures 26-30, the suturing steps are set forth as below:
1) suturing the valve leaflet 2 to the upper portion 71 (i.e. the inner side covering) of the covering 7 by a method of reciprocating suturing, as shown in figure 26.
2) bending / curling the covering 7 to a barrel shape and suturing its two ends together, as shown in figure 27.
3) folding the lower portion 72 (i.e. the outer side covering) of the covering 7 upwardly to overlap and align with the upper portion 71 (i.e. the inner side covering) of the covering 7, thus forming the inner and outer side coverings, as shown in figure 28.
4) placing the valve frame 1 between the inner and outer side coverings, as shown in figure 29.
5) suturing the upper edges of the inner and outer side coverings together by a method of edge locking suturing, to form the heart valve bioprosthesis 400, as shown in figure 30.

Herein, the valve auxiliary structure are made of biomaterials wherein the inner portion(s) inside the valve auxiliary structure (such as the covering and the suturing edge) may be made of other materials and be covered at its outer face with a layer of biomaterial, and such variation is also within the scope of the present invention.

In the heart valve bioprosthesis of the present invention, in addition to the valve leaflet made of the biomaterial, its auxiliary structure (the covering, the suturing ring, etc.) is also substantially made of biomaterials. If the terylene cloth used for suturing the covering and the suturing edge in the prior traditional heart valve bioprosthesis is not under consideration, the heart valve auxiliary structure of the present invention can be considered as being made of biomaterials only. The applicant gave up the regular means of traditionally using the terylene cloth for the covering and the suturing edge (just as mentioned in the background herein, the terylene cloth has many advantages, and thus with the teaching of the prior art, those skilled in the art generally use only the terylene cloth to make the covering and the suturing edge in manufacturing of the heart valve bioprosthesis with stent), used only the biomaterial to directly contact the blood, thus can decrease the possibility of thrombus or bacterium attachment thereto.

In the heart valve bioprosthesis of the present invention, the valve frame is integrally formed (one-step forming) using a material having high elasticity and toughness, such integral design (made into one piece) results in that the heart valve bioprosthesis of the present invention not only has the advantages of traditional valves with stent (valve frame) (such as precise positioning and easy implantation), but also has the advantages of traditional valves without stent (valve frame) (such as excellent compliance), also, it not only eliminates the disadvantages of traditional valves with stent (valve frame) (such as high strength, high hardness, complicated and risky valve frame structure), but also eliminates the disadvantages of traditional valves without stent (valve frame) (such as difficult positioning during implantation). It is made by simple processing, has an excellent compliance with the cardiac tissue, and has a prolonged valve service life. It can be precisely positioned during an implantation operation, and is easy for the surgeons to use and handle. Also, the valve designed according to the present invention is decreased in height by about 15-18% compared with the prior heart valve bioprosthesisavailable in the market, thus lowering the risk of a relatively large height of the prior heart valve bioprosthesis.

In addition, in the present invention, the valve leaflet, the suturing edge and the covering are sutured in a new cutting state and suturing method(s) such that the valve suturing process is standardized. Specifically, the sites for suturing are preset with holes, thus first establishing standardization of needle spacing during suturing such that the suturing process is more concise, more precise and more standardized. Four suturing methods, i.e. the reciprocating suturing, the edge locking suturing, the S type suturing, and the spiral suturing, are used, thus ensuring the firmness of suturing, such that it is more excellent and reliable in performance than similar products.

The preferred embodiments of the present invention are described in detail as above. However, it should be understood that upon reading the above teaching content of the present invention, those skilled in the art can make changes or modifications to the present invention. These equivalent solutions will also fall within the protection scope defined by the claims as attached in the present application.

## Claims

1. A heart valve bioprosthesis, **characterized in that** the heart valve bioprosthesiscomprises: a valve frame, a valve leaflet and a valve auxiliary structure, wherein the valve leaflet is connected to the valve auxiliary structure, the valve auxiliary structure is connected to the valve frame, and the valve leaflet and the valve auxiliary structure are made of biomaterials.

2. The heart valve bioprosthesis according to claim 1, **characterized in that** the valve auxiliary structure comprises a suturing edge and a covering, the valve leaflet is connected to the covering, and the covering and the suturing edge are fixed directly or indirectly on the valve frame.

3. The heart valve bioprosthesis according to claim 2, **characterized in that** the covering comprises an inner side covering and an outer side covering, wherein the valve leaflet is connected to the inner side covering, and the valve frame is placed within a space surrounded by the inner side covering and the outer side covering.

4. The heart valve bioprosthesis according to claim 2, **characterized in that** each of the valve leaflet, the suturing edge and the covering is preset with a suturing mark such that in suturing, the heart valve bioprosthesis is prepared by suturing along the suturing mark.

5. The heart valve bioprosthesisaccording to claim 4, **characterized in that** a distance between two adjacent ones of the suturing marks is 0.5 - 3mm.

6. The heart valve bioprosthesis according to claim 4, **characterized in that** the suturing mark is a suturing hole.

7. The heart valve bioprosthesis according to claim 1, **characterized in that** the valve frame is integrally formed.

8. The heart valve bioprosthesis according to claim 1, **characterized in that** the valve frame is made of an elastic material.

9. The heart valve bioprosthesis according to claim 1, **characterized in that** the valve frame is made of polyformaldehyde (POM), polyetheretherketone (PEET), polysulfone (PSF), Co-based alloy, Ti-based alloy, or Ni-Ti alloy.

10. The heart valve bioprosthesis according to claim 1, **characterized in that** the valve auxiliary structure is made of an animalpericardium, and the valve leaflet is made of an animalpericardium or of an aortic valve leaflet of a pig.

11. The heart valve bioprosthesis according to claim 10, **characterized in that** the animalpericardium is a pericardium of a cow/ox, a horse, a pig, a sheep/goat, a donkey, or a mule.

12. A heart valve bioprosthesis, **characterized in that** the heart valve bioprosthesis comprises: a valve frame, a valve leaflet and a valve auxiliary structure, the valve leaflet is connected to the valve auxiliary structure, the valve auxiliary structure is connected to the valve frame, wherein the valve auxiliary structure is preset with a suturing mark such that in suturing, the heart valve bioprosthesis is prepared by suturing along the suturing mark.

13. The heart valve bioprosthesis according to claim 12, **characterized in that** the valve frame is integrally formed.

14. The heart valve bioprosthesis according to claim 12, **characterized in that** the valve frame is made of an elastic material.

15. The heart valve bioprosthesis according to claim 12, **characterized in that** the valve auxiliary structure comprises a suturing edge and a covering, the valve leaflet is connected to the covering, and the covering and the suturing edge are fixed directly or indirectly on the valve frame.

16. The heart valve bioprosthesis according to claim 15, **characterized in that** the covering comprises an inner side covering and an outer side covering, wherein the valve leaflet is connected to the inner side covering, and the valve frame is placed within a space surrounded by the inner side covering and the outer side covering.

17. The heart valve bioprosthesis according to claim 15, **characterized in that** each of the valve leaflet, the suturing edge and the covering is preset with a suturing mark such that in suturing, the heart valve bioprosthesis is prepared by suturing along the suturing mark.

18. The heart valve bioprosthesis according to claim 12, **characterized in that** the valve leaflet and the valve auxiliary structure are made of biomaterials.

19. The heart valve bioprosthesis according to claim 12, **characterized in that** the elastic material is polyformaldehyde (POM), polyetheretherketone (PEET), polysulfone (PSF), Co-based alloy, Ti-based alloy, or Ni-Ti alloy.

20. The heart valve bioprosthesis according to claim 12, **characterized in that** the suturing mark is a suturing hole.

21. Amanufacturingmethod ofa heart valve bioprosthesis, **characterized in that** the manufacturing method comprises the following steps:
a) providing a valve frame, a valve leaflet, a suturing edge and a covering;
b) presetting suturing marks to the valve leaflet, the suturing edge and the covering;
c) along the suturing marks, suturing the valve leaflet to the covering, and fixing the sutured valve leaflet and covering to the valve frame;
d) fixing the suturing edge to the valve frame to form the heart valve bioprosthesis.

22. The manufacturingmethod according to claim 21, **characterized in that** the covering comprises an inner side covering and an outer side covering, and the step c) comprises: suturing the valve leaflet to the inner side covering and suturing the outer side covering to the inner side covering to form upper and lower edge coverings.

23. Themanufacturing method according to claim 22, **characterized in that** the step c) comprises: placing the valve frame into a space surrounded by the inner side covering and the outer side covering.

24. The manufacturingmethod according to claim 22, **characterized in that** the step c) comprises: suturing the valve leaflet to a top edge of the inner side covering and suturing a top edge of the outer side covering to the top edge of the inner side covering to form the upper edge covering; and the step c) comprises: suturing a lower edge of the inner side covering to a lower edge of the outer side covering to form the lower edge covering, thus enclosing the valve frame into a space surrounded by the inner side covering, the outer side covering and the upper and lower edge coverings.

25. The manufacturingmethod according to claim 21, **characterized in that** the valve frame comprises a valve ring and a valve ridge which are integrally formed; and the step d) comprises: suturing the suturing edge to an outer side edge of the valve ring.

26. The manufacturingmethod according to claim 21, **characterized in that** the suturing mark is a suturing hole.
